# NEW EUROPEAN PATENT SPECIFICATION

(11) **EP 3 700 384 B2**
(45) Date of publication and mention of the opposition decision: **23.07.2025**
(45) Mention of the grant of the patent: 06.07.2022
(21) Application number: 18804692.4
(22) Date of filing: 24.10.2018
(51) Int. Cl.: A44B 18/00, A61F 13/56, A61F 13/62

(54) **PRECURSOR WEB FOR RECLOSABLE FASTENER HOOKS AND METHODS OF MAKING**
VORLÄUFERBAHN FÜR WIEDERVERSCHLIESSBARE VERSCHLUSSHAKEN UND VERFAHREN ZU DEREN HERSTELLUNG
BANDE PRÉCURSEUR POUR CROCHETS DE FIXATION REFERMABLES ET PROCÉDÉS DE FABRICATION

(30) Priority: 27.10.2017 US 201762577907 P
(43) Date of publication of application: 02.09.2020
(73) Proprietor: Gottlieb Binder GmbH & Co. KG, 71088 Holzgerlingen (DE)
(72) Inventor: HERTLEIN, Thomas, D-41453 Neuss (DE); GORMAN, Michael R., Saint Paul, Minnesota 55133-3427 (US); PARISEAU, Timothy P., Saint Paul, Minnesota 55133-3427 (US)
(74) Representative: Kohler Schmid Möbus Patentanwälte
(86) International application number: PCT/IB2018/058305
(87) International publication number: WO 2019/082103

(56) References cited:
- WO-A1-2011/163193
- WO-A1-2013/040156
- JP-A- 2014 209 992
- US-A- 5 679 302
- US-A1- 2003 106 188
- US-A1- 2003 182 776
- US-A1- 2010 306 969

## Description

### Field

The present application relates to webs having precursors for producing mechanical fasteners, to tools for making precursors and precursor webs, and to mechanical fasteners made from the precursors and webs containing the fasteners. The mechanical fasteners are suitable for use in releasable closure systems, for example, but not limited to fastener systems for diapers.

### Background

Releasable closure systems, also called 'hook and loop fasteners' include a plurality of closely spaced upstanding projections with loop-engaging heads, also called 'hook members'. The hook members can releasably engage with woven, nonwoven, or knitted loops, the corresponding 'loop members'. Mechanical fastener systems are used to provide releasable attachments in numerous applications, in particular fastener systems for disposable absorbent articles like diapers, napkins etc. Hook structures of various shapes can be made from thermoplastic materials by molding, for example as described in U. S. Patent No. 3,594,865. Alternatively, hook members may be formed from precursors. The precursors are also formed by molding of a thermoplastic resin yielding a web containing a plurality of precursors on a backing. The web is then subjecting to a capping step where the precursors are shaped into hooks, resulting in a plurality of fasteners on the backing. Such methods are described, for example, in international patent application Nos. WO 94/23610 and 92/04839. Other technologies for making mechanical fasteners are described, for example, in US 7,198,743 B2.

There is a need for providing webs of fasteners on thin backings because such webs are mechanically more flexible and light weight. In WO2013/040156 hook members are described on thin backings. The hook members are prepared from precursors by capping. The backing was then thinned in a subsequent stretching step. Stretching, however, is an additional process step and may also lead to mechanical tensions in the backing and to a lower area density of the fasteners, which may not always be desired.

US 2010/0306969-A1 relates to a sticking closure piece comprising a support and a plurality of hooking means which are monolithically arranged on the support. Each hooking means is provided with a stem that is connected to the support, and an enlarged top part which is located at the external end of the stem. The top part is substantially flat in the region of the face thereof. The edge of the top part has a peripheral projection which points in the direction of the support and the circumferential shape of which is not rotationally symmetrical from a vertical view of the top part. The peripheral projection along the circumference of the top part extends in an irregular manner towards the support. The invention also relates to a method for producing a sticking closure piece.

There is a need for alternative methods for making mechanical fastener webs. Advantageously such methods allow to produce fasteners of various shapes without requiring thick backings.

### Summary

In the following there are provided webs containing precursors that have a greater base width than height. The precursors can be removed from the molds using little force and the precursors webs can have a thin backing layer. The precursors can be converted into mechanical fasteners.

In the following there is provided a method of making a web (40) comprising: providing a web (10) of precursors (18) for mechanical fasteners (48), the web (10) comprising at least one backing layer (30) and a plurality of upstanding precursors (18) for mechanical fasteners arranged on the backing layer (30) and integral with the backing layer (30), wherein each precursor (18) of the plurality of precursors has a distal end (12) and a proximal end (16) by which the precursor (18) is connected to the backing layer (30), and wherein the base width (22) at the proximal end (16) of the precursor is greater than the height (24) of the precursor (18), and wherein the precursors (18) taper towards their distal ends (12), and wherein the backing layer (30) has a thickness (32) of from about 10 µm to about 75 µm.

In another unclaimed aspect there is provided a tool (80) for making the web (10) wherein the tool (80) comprises a mold surface (79) having a plurality of cavities (78) having the inverse shape of the precursors (18).

In a further unclaimed aspect there is provided a method of making the tool (80) comprising depositing material on a surface or removing material from a surface or both to create a plurality of cavities (78) having the inverse shape of the precursors (18) to provide a mold surface (79).

In yet another unclaimed aspect there is provided a method of making the web (10) comprising
- providing the tool (80);
- feeding a resin into the cavities (78) and onto the mold surface (79) of the tool to form a backing covering the cavities (78) and having a thickness of from about 10 µm to about 75 µm;
- solidifying the resin within the cavities (78) and on the mold surface (79) to form the precursors (18) arranged on the backing layer (30);
- stripping the backing (30) with the integrally formed precursors (18) from the mold surface (79);
- optionally winding up the resulting web (10) into a roll.

The method according to the invention also comprises the following steps: creating a web (40) of mechanical fasteners by subjecting the precursors (18) to shaping to form the fasteners (48), the web (40) comprising at least one backing layer (30) and a plurality of upstanding mechanical fasteners (48) arranged on the backing layer (30) and integral with the backing layer (30), wherein each of the mechanical fasteners (48) of that plurality has (i) head portion (42) comprising the distal end (44) of the fastener (48) and (ii) a stem portion linking the head portion (42) of the fastener (48) with the backing layer (30) and comprising the proximal end (50) by which the fastener (48) is linked with the backing layer (30), wherein the stem portion is tapered and the width (52) of the head portion (42) is greater than the width (58) of the stem immediately underneath the head portion (42) such that the head portion (42) forms an overhang and wherein the width (52) of the head portion (42) is equal or less than the base width (54) of the fastener (48) at the proximal end (50) and wherein the base width (54) is greater than their height (26) of the fastener (48), and wherein the thickness (32) of the backing layer (30) is from about 10 µm up to about 75 µm, wherein head portions of the upstanding precursors (18) of the web of precursors (18) are deformed using heat and/or pressure, wherein deforming comprises contacting distal tips of the upstanding precursors (18) of the web of precursors (18) with a heated surface and/or contacting the distal tips of the upstanding precursors with a surface that is not heated, wherein if the surface is not heated, the defomation is carried out with pressure and without heating, wherein the head portion forms an overhang on each side of the stem.

According to the invention there is provided a method of making the web (40) of fasteners comprising
- providing the web (10) of precursors;
- creating the web (40) of fasteners by subjecting the precursors (18) to shaping to form the fasteners (48).

In yet another unclaimed aspect there is provided an absorbent article (100) comprising the web (40) of fasteners.

In a further unclaimed aspect there is provided a method of making an absorbent article (100) comprising (i) providing an absorbent article, (ii) connecting the web (40) of fasteners to the absorbent article (100).

### Brief description of the drawings

The disclosure may be more completely understood in consideration of the following detailed description of various embodiments of the disclosure in connection with the accompanying drawings, in which:
FIG. 1 A is a cross-sectional view of an exemplary precursor for a mechanical fastener as disclosed herein on a backing layer.
FIG. 1 B is the top view of the precursor if FIG 1A.
FIG. 2A is a cross-sectional view of a mechanical fastener on a backing as disclosed herein.
FIG. 2B is a top view of the mechanical fastener of FIG. 2A.
FIG. 3 is a schematic representation of a diaper containing fasteners as disclosed herein.
FIG. 4 is a schematic illustration of the process of making a web having precursors according to the present disclosure.
FIG. 5 is a schematic illustration of the process according to the present disclosure of converting a web containing a plurality of precursors into a web containing a plurality of mechanical fasteners.

### Detailed description

It is understood that the drawings and the following detailed description are for illustrative purposes only and should not be read in a manner that would unduly limit the scope of this disclosure. Reference will now be made in detail to embodiments of the disclosure, one or more examples of which are illustrated in the drawings. Features illustrated or described as part of one embodiment can used with other embodiments to yield still a further embodiment. It is intended that the present disclosure include these and other modifications and variations.

Also, it is to be understood that the phraseology and terminology used herein is for the purpose of description and should not be regarded as limiting. Contrary to the use of "consisting", the use of "including," "containing", "comprising," or "having" and variations thereof is meant to encompass the items listed thereafter as well as additional items. The use of "a" or "an" is meant to encompass "one or more". Any numerical range recited herein is intended to include all values from the lower value to the upper value of that range and including the end points of the range. For example, a concentration range of from 1% to 50% is intended to be an abbreviation and to expressly disclose the values 1% and 50% and the values between the 1% and 50%, such as, for example, 2%, 40%, 10%, 30%, 1.5%, 3.9% and so forth. Reference numerals are used for illustration only.

In this disclosure webs of precursors are provided. The webs of precursors according to the present disclosure contain a plurality of upstanding posts arranged on a backing layer. The posts are rather flat but rather wide. Due to that shape the precursors can be easily removed from molds and therefore can be obtained on thin backings. Mechanical fasteners on thin backings can be prepared from these precursors, by shaping the precursors to provide a loop-engaging head region, by creating an overhang in the head region, for example by capping. Therefore, webs of fasteners on thin backings can be obtained from the web of precursors without requiring stretching steps - although stretching may still be done. The fasteners resulting from the precursors have low height and small head regions but still have similar shear and peel strength behavior than commercial fasteners.

Therefore, the posts are referred to herein also as "precursors" or "precursors for mechanical fasteners".

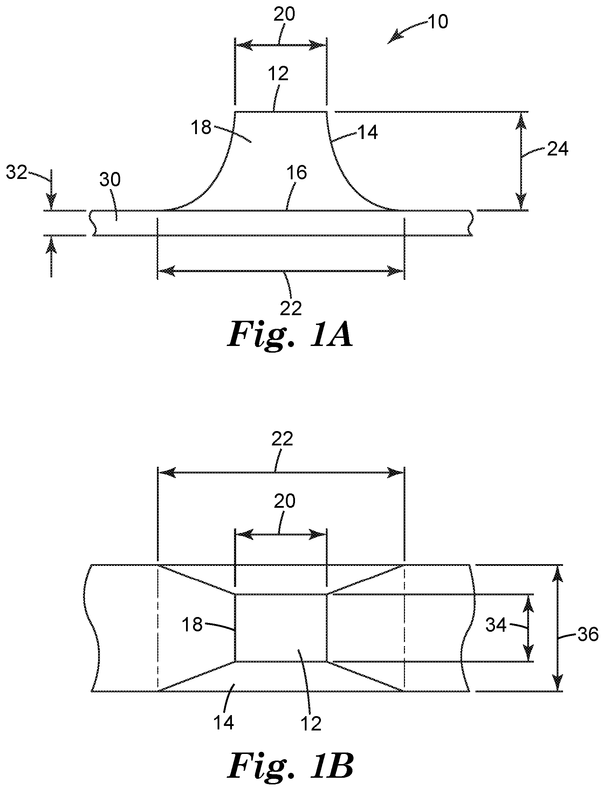

The plurality of precursors will now be described in greater detail by referring to Fig. 1A and Fig. 1B which illustrate a precursor of the web of precursors according to the present disclosure. Each precursor (18) of that plurality is connected by its proximal end (16) with the backing layer (30) and forms an integral part with the backing layer (30). The web may contain a single backing layer (30) as is illustrated in Fig. 1 A but the web may also contain additional layers connected to the bottom side of the backing layer (30), i.e. the side opposite to the where the precursors are arranged. For example, but not limited thereto, one or more adhesive layer and release liners may be attached to the backing layer (30). The precursors (18) are upstanding protrusions, e.g. posts, from the backing layer (30). As referred to herein the term 'upstanding' means the precursors (18) either protrude perpendicularly from the backing layer (30) or they protrude substantially perpendicularly from the backing, i.e., they protrude from the backing layer (30) at an angle of 90°C +/-30°C.

The precursor (18) comprises a head portion that includes the distal end (12) of the precursor. The precursor also has a base portion that includes the proximal end (16) of the precursor. The head portion may begin at the distal end (12) and continues towards the proximal end (16). It may form from 1% to about 50% of the height of the precursor above the backing. The base portion starts with the proximal end (16) and extends towards the distal end (12) of the precursor. It may comprise up to 50% of the height of the precursor. Between head portion and base portion there may be one or more than one middle portions.

The height (24) of the precursors is the height of the posts above the backing layer (30), i.e. the distance between the distal end (12) and the proximal end (16) of the precursors (18) as is illustrated in FIG. 1A. In case the distal end (12) is not even or flat, and there are several distances between distal and proximal ends, the height (24) refers to the maximum distance. The precursors of the present disclosure may have a height (24) of about 100 µm to about 1000 µm, or from 151 µm to 495 µm, preferably from about 200 µm to about 400 µm.

For the purpose of this disclosure, all dimensions of the upstanding precursors or fasteners and backing layers described herein are measured by optical microscopy.

The precursors have a width at their base (base width) that is greater than their height (24). The base width (22) is the width measured at the proximal end (16). The term "width" should be understood to include the diameter of the post (18) with a circular cross-section. When the post has more than one width dimension (e.g. when the post (18) is of rectangular or elliptical shape, the term "width" refers to the largest width dimension, e.g. the largest axis or diameter - as illustrated in FIGs. 1A and 1B. As illustrated in FIG 1B the post is of rectangular shape and has at the basis a length (22) and a length (36). The basis width (22) is the length of the longest axis, which as illustrated in FIG 1B corresponds to the length (22). The precursors (18) may have a width (22) at their base of from about 105 µm to about 1,010 µm, preferably from about 220 µm to about 900 µm, more preferably from 300 µm to about 600 µm. The ratio of base width (22) to height (24) of the precursors (18) may include ratios from about 10:1 to 1.1:1.0. Suitable ratios of base width to height include ratios of from 5:1 to 1.2:1.0 or from 3.5:1.0 to 1.5:1.0.

The precursors (18) may have a variety of cross-sectional shapes. For example, the cross-sectional shape of the post may be a polygon (e.g., square, rectangle, hexagon, or pentagon), which may be a regular polygon or not, or the cross-sectional shape of the post may be curved (e.g., round or elliptical). The precursors (18) may have a symmetric or non-symmetric shape. For example, when viewed from the top (illustrated in FIG. 1B) the shape of the posts (18) may correspond to a polygon (e.g., square, rectangle, hexagon, or pentagon), which may be a regular polygon or not, or the shape of the post viewed from top may be curved (e.g., round or elliptical). The posts (18) taper and decrease in size from the proximal ends toward the distal ends (as illustrated in FIG. 1A and 1B). The precursors (18) may be tapered such that their width decreases from their base (22) towards their distal ends (12). A tapered post (18) is illustrated in FIG. 1A where the precursor (18) has flanks (14) which continuously incline towards the distal end (12). The flanks (14) illustrated in FIG 1A and 1B are continuously curved, but tapered forms where the width does not decrease continuously are also suitable. For example, the width may decrease discontinuously and the flanks (14) would then contain straight and curved sections. The decrease in width may take place in discrete intervals interrupted by one or more section where the width does not decrease and remains constant. In a preferred embodiment the base width (22) of the precursor (18) is greater than the width at the distal end (20). The posts are tapered because they can be easier demolded. Preferred ratios of width (22) at the base to width (20) at the distal end include, but are not limited to, ratios from about 5 to 1 to about 1.1 to 1.0. Like with the basis width also the width at the distal end is the longest axis. For example, as illustrated in FIG 1B the post is of rectangular shape of the distal end and has the length (20) and the length (34). The width at the distal end of the post illustrated in FIG 1B corresponds to the length (20).

It is an advantage of the precursors according to the present disclosure that due to their shape they can be demolded easily and requiring only little force. This means thinner resin layers may be used when demolding the precursors and therefore the resulting webs can have precursors arranged on a thin backing layer. The backing layer (30) has a thickness (32) from about 10 µm up to about 75 µm, preferably from about 15 µm up to about 70 µm, more preferably from about 21 µm up to about 65 µm, or from about 21 µm up to about 50 µm. In one embodiment, the backing layer (30) has a thickness (32) from about 10 µm up to about 19 µm. A low thickness of the backing layer, or the web, is useful for saving raw materials. Additionally, backing layers or webs of small thickness have an increased mechanical flexibility and are of lower weight. For example, webs with thin backing layers can be easier wound up into rolls and can be transported more easily because they have a reduced weight. Moreover, increased flexibility helps to provide for a good fit of the web on the articles to which they are to be applied.

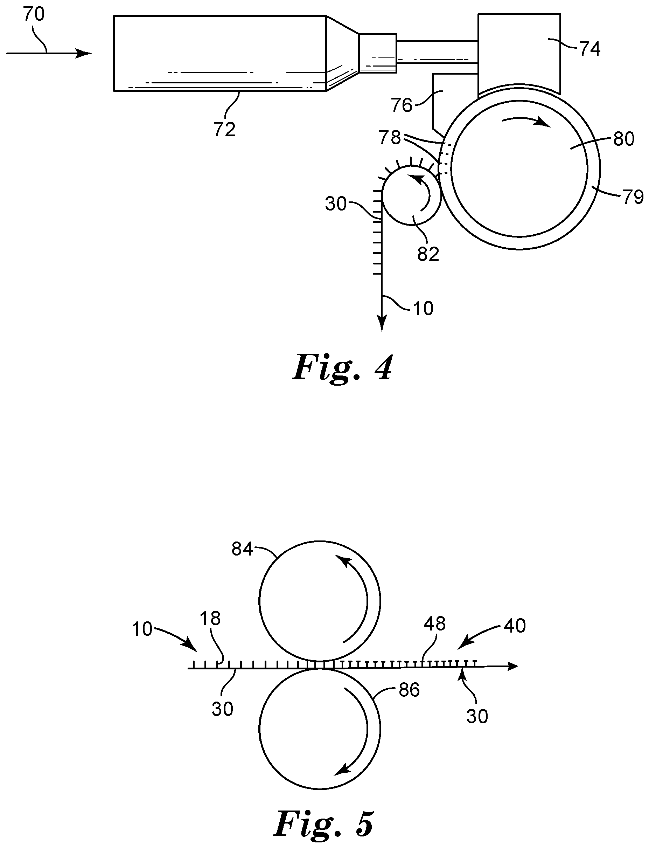

In the precursors for mechanical fasteners according to the present disclosure the backing layer and the upstanding posts are integral (that is, formed at the same time as a unit, unitary). In some embodiments, the backing layer and the precursors are made from the same thermoplastic material. The thermoplastic backing is typically in the form of a sheet or web that may have an essentially uniform thickness with the upstanding precursors directly attached to the thermoplastic backing. Upstanding posts on a backing can be made, for example, by conventional extrusion through a die and cast molding techniques as illustrated in FIG. 4. A thermoplastic material (70) is fed, typically via the die (74) of an extruder (72) onto a continuously moving mold surface (79) with cavities (78) having the inverse shape of the upstanding posts (18). The height (24) of the posts (18) is determined by the depth of the cavities (78). The thermoplastic material can be passed between a nip formed by two rolls or a nip between a die face and roll surface, with at least one of the rolls (80) having the cavities (i.e., at least one of the rolls is a tool roll (80)). Pressure provided by the nip forces the resin into the cavities. In some embodiments, a vacuum can be used to evacuate the cavities for easier filling of the cavities. The nip has a gap that is typically sufficiently big such that a coherent backing is formed over the cavities. The nip gap and also the throughput of the extruder and the speed of the mold surface can be adjusted to achieve the thickness of the backing layer (30) as described herein. An endless metal or polymer belt or a metal belt coated with polymer may be useful to provide even pressure in the nip. The mold surface and cavities can optionally be air or water cooled before stripping the integrally formed backing (30) and upstanding posts (18) from the mold surface (79) such as by a stripper roll (82).

Suitable tool rolls (80) can be made, for example, by forming (e.g., by computer numerical control with drilling, photo etching, using galvanic printed sleeves, laser drilling, electron beam drilling, metal punching, direct machining, or lost wax processing) a series of holes having the inverse shape of the upstanding posts into the cylindrical face of a metal mold or sleeve. Other suitable tool rolls include those formed from a series of plates defining a plurality of post- forming cavities about its periphery such as those described, for example, in U.S. Pat. No. 4,775,310 (Fischer). Cavities may be formed in the plates by drilling or photoresist technology, for example. Still other suitable tool rolls may include wire-wrapped rolls, which are disclosed along with their method of manufacturing, for example, in U.S. Pat. No. 6,190,594 (Gorman et al.). The exposed surface of the mold, sleeve, plate, or wire may be coated to impart surface properties such as increased wear resistance, controlled release characteristics, and controlled surface roughness. The coating, if present, is preferably selected so that the adhesion of the thermoplastic material to the tool roll is less than the cohesion of the thermoplastic material at the time of the removal of the thermoplastic backing from the tool roll.

Another exemplary method for forming a thermoplastic backing with upstanding posts includes using a flexible mold belt defining an array of upstanding post-shaped cavities as described in U.S. Pat. No. 7,214,334 (Jens et al.). The mold belt is trained about first and second rolls, and a source of molten thermoplastic material is arranged to deliver the thermoplastic to the mold belt. The apparatus is constructed to force the plastic resin into the upstanding post-shaped cavities of the belt under pressure in a gap to mold the array of upstanding posts while forming the thermoplastic web layer.

Many thermoplastic materials are useful for precursors of mechanical fasteners according to the present disclosure. Suitable thermoplastic materials for the thermoplastic backing with upstanding elements include polyolefin homopolymers such as polyethylene and polypropylene, copolymers of ethylene, propylene and/or butylene; copolymers containing ethylene such as ethylene vinyl acetate and ethylene acrylic acid; polyesters such as poly(ethylene terephthalate), polyethylene butyrate and polyethylene napthalate; polyamides such as poly(hexamethylene adipamide); polyurethanes; polycarbonates; poly(vinyl alcohol); ketones such as polyetheretherketone; polyphenylene sulfide; poly(acrylonitrile-butadiene-styrene); plasticized polyvinylchlorides; and mixtures thereof. Typically, the thermoplastic is a polyolefin (e.g., polyethylene, polypropylene, polybutylene, ethylene copolymers, propylene copolymers, butylene copolymers, and copolymers and blends of these materials). The various thermoplastic materials described above can be formulated into a master batch having a desired property (e.g., color); however, the presence or absence of dyes, pigments, or other colorants are not essential to this disclosure. Suitable thermoplastic materials that allow good filling of small-sized cavities have typically a low viscosity (e.g. a polypropylene with a melt flow rate of 25 g/10min or higher). Suitable thermoplastic materials that allow fast solidification of the melt after filling of the cavities and by such resulting in a short time for deforming, contain typically small amounts of additives the increase rate of crystallization of the polymer (e.g. nucleating agents).

In some embodiments, the thermoplastic backing with upstanding elements can be made from a multilayer or multi-component melt stream of thermoplastic materials. This can result in elements formed at least partially from a different thermoplastic material than the one predominately forming the backing. A multilayer or multi-component melt stream can be formed by any conventional method. A multilayer melt stream can be formed by a multilayer feedblock, such as that shown in U.S. Pat. No. 4,839,131 (Cloeren). A multicomponent melt stream having domains or regions with different components could also be used. Useful multicomponent melt streams could be formed by use of inclusion coextrusion die or other known methods (e.g., that shown in U.S. Pat. No. 6,767,492 (Norquist et al.).

In a non-claimed aspect the present disclosure also refers to a tool for making a web containing the precursors as described herein. The tool has a surface containing a plurality of cavities shaped as negatives of the plurality of precursors according to the present disclosure. Presursors of shape and dimensions as described herein can be obtained by filling a moldable resin, typically in flowable form into the cavities. The resin is then allowed to solidify in the cavities, for example by cooling, or by curing it into a solid form. Removing the resin from the cavities yields the precursors. If the resin is filled in the cavities and also over the mold surface a web containing the cavities is obtained after stripping off the resin from the cavities and the mold surface. By controlling the thickness of the resin layer on the mold surface the thickness of the resulting backing layer (30) can be controlled. The thickness can be low and stretching of the resulting web to obtain thinner backings may be carried out but is not necessary.

The tool may be a cylinder having the cavities on its surface. Alternatively, the mold can contain several parts which may be attachable or can be removed from each other, for example the surface of the mold containing the cavities may be mounted on a carrier and removed from the carrier. Molds, or mold surfaces containing the cavities can be made by removing materials from a surface (etching, cutting, milling, drilling) or by method adding material (electro-plating) to create the desired cavities. Techniques known in the art may be used. Such methods may involve forming the required cavities by chemical etching, electro-plating, laser-etching or e-drilling as described, for example, in US patents Nos 5,900,350 and 6,289,766.

### Mechanical Fasteners:

The precursors formed upon exiting the cavities of the mold typically do not have loop-engaging head regions, for example caps, but they are subsequently formed in a subsequent shaping step, for example, as illustrated in Fig. 5. A useful shaping step includes capping in a nip formed by two rollers (84 and 85). The capping method includes deforming the head portions of the upstanding posts using heat and/or pressure. The heat and pressure, if both are used, could be applied sequentially or simultaneously. In some embodiments, deforming comprises contacting the distal tips of the upstanding posts with a heated surface. The heated surface may be a flat surface or a textured surface. Capping methods are described, for example in US Pat. Nos. 5,607,635 (Melbye et al.) 6,708,378 (Parellada et al.) or 5,868,987 (Kampfer et al.). In some embodiments, wherein the thermoplastic backing with upstanding precursors is a web of indefinite length, deforming the distal tips of the posts (precursors) to form caps includes moving the web in a first direction through a nip having a heated surface member and an opposing surface member such that the heated surface member contacts the distal tips. In these embodiments, the heated surface may be, for example, a capping roll. In some embodiments, the surfaces used to contact the distal tips may not be heated. In these embodiments, the deformation is carried out with pressure and without heating. In some embodiments, the heated surface may be a heated roll opposite a curved support surface forming a variable nip having a variable nip length as described, for example, in U. S. Pat. No. 6,368,097 (Miller et al.). The curved support surface may curve in the direction of the heated roll, and the heated roll may include a feeding mechanism for feeding the thermoplastic backing with upstanding posts through the variable nip to compressively engage the web between the heated roll and the support surface. In some embodiments, heating is carried out below a melt temperature of the distal tips. When the thermoplastic material used to form the upstanding posts is a copolymer (e.g., copolymers of ethylene and propylene), the distal tips may have more than one melt temperature. In these embodiments, "below a melt temperature of the distal tips" means below at least one of the melt temperatures.

In some embodiments, distal caps of the upstanding fastening elements are reshaped after they are formed. For example, passing a thermoplastic backing having upstanding capped posts through a gapped nip of a heated rubber roll and a backup roll causes the overhanging portions of the distal cap, that extend beyond the post, to be pushed down toward the backing. This process is described in U.S. Pat. No. 6,132,660 (Kampfer).

In addition to the continuous methods described above, it is also envisioned that thermoplastic backings having upstanding fastening elements can be prepared using batch processes (e.g., single piece injection molding). The thermoplastic backing may have any suitable dimension, but length (L) and width (W) dimensions of at least 10 centimeters may be useful.

The present disclosure also provides fastener webs, i.e. webs containing a plurality of upstanding posts arranged on a backing layer of the web wherein the posts contain a loop-engaging head region. The webs of fasteners can be obtained by subjecting the precursors described above to a shaping step where the head regions of the precursors are formed into loop-engaging structures, for example by capping.

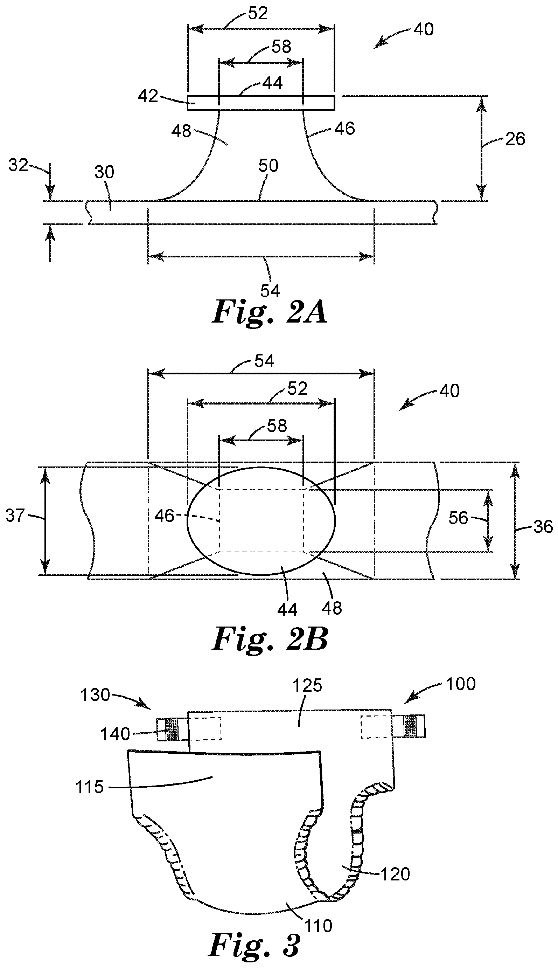

The plurality of the fasteners will now be described in greater detail by referring to Fig. 2A and Fig. 2B which illustrate a fastener of the web of fasteners according to the present disclosure. Each fastener (48) of that plurality is connected by its proximal end (50) with the backing layer (30) and forms an integral part with the backing layer (30). The web of fasteners (40) may contain a single backing layer (30) as is illustrated in Fig. 2 A but the web may also contain additional layers connected to the bottom side of the backing layer (30), i.e. the side opposite to the where the precursors are arranged. For example, but not limited thereto, one or more adhesive layer and release liners may be attached to the backing layer (30). The fasteners (48) are upstanding posts from the backing layer (30) and contain a head portion (42) and a stem. The stem connects the head portion (42) with the backing layer (30) and comprises the proximal end (50) of the fastener (48). The term 'upstanding' with respect to the fasteners has the same meaning as used with respect to the precursors. The head (42) includes the distal end (44) of the fastener.

The fasteners of the present disclosure may have a height (26) that is smaller than the height of the precursors from which they originate, for example due to the capping. Typically, the fasteners have a height of from about 90 µm to about 900 µm, preferably from about 180 µm to about 360 µm. The height (26) of the fasteners is the height of the fasteners above the backing, i.e. the maximum distance between the distal end (44) and the proximal end (50) of the fasteners (48) as illustrated in FIG. 2A.

The width at the base (base width, (54)) of the fasteners (48) is greater than their height (26). The fasteners typically have the same width at their base as their precursors. They may have a width at their base of from about 105 µm to about 1010 µm, preferably from about 220 µm to about 900 µm, more preferably from 300 µm to about 600 µm. Like the precursors the term "width" should be understood to include the diameter of the stem with a circular cross-section. When the post has more than one width dimension (e.g. when the stem is of rectangular or elliptical shape, the term "width" refers to the largest width dimension, e.g. the largest axis or diameter - as illustrated in FIGs. 2A and 2B. The ratio of base width (54) to height (26) of the fasteners may be greater than the corresponding ratio of their precursors. Typical ratios of base width (54) to height (26) may include ratios from about 10.5:1.0 to 1.2:1.0. Suitable ratios of base width to height include, but are not limited to, ratios of from 6:1 to 1.4:1.0 or from 3.8:1.0 to 1.6:1.0.

The stems may have a variety of shapes. For example, the shape of the stems may be a polygon (e.g., square, rectangle, hexagon, or pentagon), which may be a regular polygon or not, or the cross-sectional shape of the stems may be curved (e.g., round or elliptical).

The stems taper and decrease in width from their proximal ends toward their distal ends (as illustrated in FIG. 2A). The stems of the fasteners may taper such that their width decreases continuously from their base width (54) as illustrated in FIG. 2A where the stem of the fastener (48) has flanks (46) which continuously incline. The flanks (46) illustrated in FIG 2A and 2B are curved, but they may also be straight or may contain straight and curved sections or sections with different curvature or different straightness.

In other embodiments the stems are tapered but the width of the stems decreases discontinuously, for example in discrete intervals interrupted by at least one interval where the width remains constant. The base width (50) of the fasteners (48) is greater than the width (58) immediately underneath their heads (42). The width (58) immediately underneath the heads (42) may be from about 100 µm to about 1000 µm, provided that the width is smaller than the width at the base (54). Suitable ratios of width at the base (54) to width (58) immediately underneath the heads (42) include ratios from about 5 to 1 to about 1.1 to 1, preferably from about 2,5 to1 to about 1,5 to 1.

Generally, the heads (42) of the upstanding fastening elements (48) have a shape that is different from their precursors. The heads (42) protrude over the immediately underlying portion of the stem (58), i.e. the heads have at least one overhang. For example, the fastening element (48) may be in the cross-sectional shape of a mushroom (e.g., with a circular or oval head enlarged with respect to the underlying part of the stem), a nail, or a T. The heads (42) have an overhang on each side of the stem (58). The extent of overhang on the two opposing sides may or may not be equal. In some of these embodiments, the fastener (48) is in the shape of a mushroom (e.g., with a circular or oval head enlarged with respect to the immediately underlying stem) or a nail. At least a portion of the overhang is in all directions although the amount of overhang may not be equal in all directions. In some embodiments the overhang extends up to 90 µm beyond the stem immediately underneath the head (42) in at least one direction. In other embodiments, the overhang is up to 85 µm, 80 µm or 75 µm. The minimum overhang may be selected, for example, based on the fiber diameter of the loop that is selected to engage with the mechanical fastener disclosed herein. In some embodiments, the overhang is at least 5 µm or at least 10 µm. The overhang may be in the range, for example, from 5 µm to 85 µm, or from 5 µm to 65 µm.

The overhang in the upstanding fastening elements disclosed herein is typically considered to be loop-engaging. The term "loop-engaging" as used herein relates to the ability of an upstanding fastening element to be mechanically attached to a loop material.

For creating an overhang the width (52) of the heads (42) of the fasteners (48) is greater than the width (58) of the stems directly underlying the heads. Since the stems are tapered, the width (58) of the stems directly underlying the head is also smaller than the width at the base (54). As illustrated in FIG 2A and FIG 2B the width (52) of the heads (42) is the longest dimension of the heads and includes a diameter in case the head is of circular or oval shape.

The heads (42) can have various shapes and may be symmetric or non-symmetric. For example, when viewed from the top (illustrated in FIG. 2B) the heads (42) may have the shape of a polygon (e.g., square, rectangle, hexagon, or pentagon), which may be a regular polygon or not, or they may be curved (e.g., round or elliptical). The surface of the heads may be flat or structured.

Typically, the fasteners elements are obtained by providing the web of precursors to a shaping step to give the head region of the precursors a loop-engaging shape. Typically, the web or the backing layer and the base portion of the precursors may not be affected by the shaping step taken to convert the web of precursors into a web of fasteners. Therefore, the web of fasteners has a backing layer (30) as described above with respect to the precursors. The backing layer has a thickness (32) between 10 µm to 75 µm, preferably from about 15 µm to about 70 µm, more preferably from about 21 µm up to about 65 µm, or from 21 µm to 50 µm. In one embodiment the backing layer (30) has a thickness (32) of from about 10 µm to 19 µm. In the mechanical fasteners according to the present disclosure the backing layer and the upstanding posts are integral (that is, formed at the same time as a unit, unitary). In some embodiments, the backing layer and the stems of the fasteners or the fasteners are made from the same thermoplastic material. The thermoplastic material typically is the same as described above for the precursors.

The web of fasteners may contain additional layers, as will be described below with respect to fastening laminates.

The web (40) of fasteners according to the present disclosure may be wound up into a roll for storage and transportation. An advantage of the webs according to the present disclosure is that webs can have very thin backings and are therefore of lower weight and can be easier stored, transported or processed.

For any of the embodiments of the mechanical fastener according to the present disclosure, the thermoplastic backing may be in the form of a roll, from which mechanical fastener patches containing a plurality of fasteners, for example, may be cut in a size appropriate to the desired application. In this application, the thermoplastic backing may also be a patch that has been cut to a desired size. In some of these embodiments, the second surface of the thermoplastic backing (i.e., the surface opposite the first surface from which the upstanding fastening elements project) may be coated with an adhesive (e.g., a pressure sensitive adhesive). In such embodiments, when the thermoplastic backing is in the form of a roll, a release liner may be applied to the exposed adhesive.

### Fastening Laminates

Fastening laminates that can be formed after joining the thermoplastic backing layer to a carrier may be useful, for example, in disposable absorbent articles. The fastening laminate may be in the form of a fastening tab that is bonded to at least one of the front waist region or the rear waist region extending outwardly from at least one of the left longitudinal edge or the right longitudinal edge of the absorbent article. In other embodiments, the fastening laminate may be an integral ear portion of the absorbent article.

In some embodiments of the mechanical fastener disclosed herein, the thermoplastic backing is not joined to a carrier, at least when it is initially formed. In other embodiments, the second surface of the thermoplastic backing (i.e., the surface opposite the first surface from which the upstanding fastening elements project) is joined to a carrier. The thermoplastic backing may be joined to a carrier, for example, by lamination (e.g., extrusion lamination), adhesives (e.g., pressure sensitive adhesives), or other bonding methods (e.g., ultrasonic bonding, compression bonding, or surface bonding). The thermoplastic backing may also be joined to a carrier during the formation of the thermoplastic backing with upstanding posts. The resulting article may be a fastening laminate, for example, a fastening tab joined to the back sheet of an absorbent article useful for joining the front waist region and the rear waist region of an absorbent article. The carrier, which in some embodiments may be joined to the second surface of the thermoplastic backing, may be continuous (i.e., without any through-penetrating holes) or discontinuous (e.g. comprising through-penetrating perforations or pores). The carrier may comprise a variety of suitable materials including woven webs, non-woven webs (e.g., spunbond webs, spunlaced webs, airlaid webs, meltblown web, and bonded carded webs), textiles, plastic films (e.g., single- or multilayered films, coextruded films, or films comprising foam layers), and combinations thereof. In some embodiments, the carrier is a fibrous material (e.g., a woven, nonwoven, or knit material). The term "nonwoven" when referring to a carrier or web means having a structure of individual fibers or threads which are interlaid, but not in an identifiable manner as in a knitted fabric. Nonwoven fabrics or webs can be formed from various processes such as melt blowing processes, spunbonding processes, spunlacing processes, and bonded carded web processes. In some embodiments, the carrier comprises multiple layers of nonwoven materials with, for example, at least one layer of a meltblown nonwoven and at least one layer of a spunbonded nonwoven, or any other suitable combination of nonwoven materials. Or, the carrier may be a composite web comprising a nonwoven layer and a dense film layer.

Fibrous materials that provide useful carriers may be made of natural fibers (e.g., wood or cotton fibers), synthetic fibers (e.g., thermoplastic fibers), or a combination of natural and synthetic fibers. Exemplary materials for forming thermoplastic fibers include polyolefins (e.g., polyethylene, polypropylene, polybutylene, ethylene copolymers, propylene copolymers, butylene copolymers, and copolymers and blends of these polymers), polyesters, and polyamides. The fibers may also be multi- component fibers, for example, having a core of one thermoplastic material and a sheath of another thermoplastic material.

One or more zones of the carrier may comprise one or more elastically extensible materials extending in at least one direction when a force is applied and returning to approximately their original dimension after the force is removed. The term "elastic" refers to any material that exhibits recovery from stretching or deformation. Likewise, "nonelastic" materials, which do not exhibit recovery from stretching or deformation, may be useful for the carrier as well.

### Fastening systems

Mechanical fasteners according to the present disclosure are useful components in fastening systems that include the mechanical fastener according to any of the above embodiments and a loop material. Although mechanical fasteners according to the present disclosure are useful with a variety of different loop materials, in some embodiments, the loop material is a low-loft loop material, which is desirable for use in fastening systems because of their low cost and low material-usage. Examples of low-loft materials include nonwoven materials, for example, made from any of the materials described above for carriers. In some embodiments, the loop material has a fiber basis weight in a range from 10 grams per square meter (gsm) to 30 gsm. Fiber basis weight is a basis weight of just the fiber in the loop material (e.g., removed from any backing). In some embodiments, the loop material has a fiber basis weight in a range from 10 gsm to 20 gsm or from 15 gsm to 20 gsm. In some embodiments, the loop material has a fiber diameter in a range from 15 micrometers to 25.4 micrometers. The loop material may have a loop height of up to 500 µm and a loop width of up to 2500 µm. In some embodiments, the loop material has a fiber basis weight in a range from 20 grams per square meter to 30 grams per square meter, and the mechanical fastener engages with the loop material with a shear strength of at least 2000 gram-force. However, in other embodiments, mechanical fasteners may be useful with loop materials having fiber basis weights in a range from 30 gsm to 50 gsm as well.

### Absorbent articles

Fastening laminates according to the present disclosure may also be useful, for example, as fastening tabs for pants-style diapers such as those described in U.S. Pat. No. 5,531,732 (Wood). In some embodiments, the absorbent article according to the present disclosure is a disposable pants-style diaper having a fibrous outer covering or back sheet that can engage with upstanding fastening elements on a fastening tab disclosed herein. The fastening tab can be located on a seam or side panel portion of the pants-style diaper such that a free end of the fastening tab is able to engage the fibrous outer covering or back sheet. The fastening tab free end is useful, for example, for adjusting the pants-style diaper's circumferential fit or size (in other words, waist fit or size) by gathering the side panel portion. The side panel portion may be free of any integrally bonded absorbent core structure in some embodiments. In some embodiments, the pants-style diaper has at least one perforation line extending from the waist opening to one of the leg openings. The perforation is generally near the side seam, may be positioned toward the front portion of the diaper, and may be parallel or non-parallel to the side seam. In some embodiments, the pants-style diaper has a pair of perforation lines, one on each side of the diaper. The perforation line(s) can either be broken before the diaper is positioned around the user's torso or while the user is wearing the diaper. The fastening tab free end can then be used to refasten the diaper so that it is snug around the user's waist. The fastening tab free end may also be useful, for example, as a disposal means when the pants-style diaper is removed from the wearer (e.g., by tearing the side panel or perforation line). The fastening tab typically remains on the side panel portion. The diaper can then be rolled into a compact form for disposal, and the fastening tab can be used to keep the diaper in a rolled form. In some embodiments, a fastening tab according to the present disclosure can be applied to a pants-style diaper in a manufacturing process by using a laminate in which separated strips of the mechanical fastener disclosed herein are laminated to a nonwoven web using any of the methods described above. In some embodiments, two strips of the mechanical fastener can be positioned on the nonwoven web with the nonwoven extending beyond the mechanical fastener strips on both sides such that there are first and second side portions and a central portion of exposed nonwoven separated by the mechanical fastener strips of the laminate. The laminate can be in the form of a roll with longitudinally extending separated mechanical fastener strips. In an efficient embodiment of the manufacturing process of a pants-style diaper, the laminate can be cut in the cross-direction to a desired width of a fastening tab and aligned with two connected diapers in a web of diaper chassis. Then, the laminate can be cut down the middle of the central portion of exposed nonwoven at or about the same time the two connected diapers are cut apart so that half the laminate is applied to one diaper and half is applied to the other diaper. Then bonding of the side seams of the diaper and bonding of the fastening tab to the side seams can be carried out simultaneously, if desired. In these embodiments, each of the first and second side portions of exposed nonwoven can advantageously serve as a fingerlift for the left and right fastening tabs of two different pants-style diapers.

The fastening laminate including the mechanical fastener disclosed herein may also be useful, for example, for absorbent articles such as sanitary napkins. A sanitary napkin typically includes a back sheet that is intended to be placed adjacent to the wearer's undergarment. The back sheet may comprise a thermoplastic backing with spaced-apart, upstanding capped posts to securely attach the sanitary napkin to the undergarment, which mechanically engages with the capped posts. The back sheet may formed with upstanding capped posts. In other embodiments, the mechanical fastener may be in the form of a strip or patch that is attached to the back sheet with adhesive or using another bonding mechanism.

In other embodiments, the mechanical fastener according to the present disclosure may be useful, for example, in absorbent pads having at least a top sheet, an absorbent core, and a back sheet, wherein the back sheet comprises the mechanical fastener. The back sheet may formed with upstanding capped posts. In other embodiments, the mechanical fastener may be in the form of a strip or patch that is attached to the back sheet with adhesive or using another bonding mechanism. The absorbent pads may be useful, for example, in adult incontinence articles, which may be in the form of an open-type diaper having the general shape such as that shown in FIG. 3 or a pants-type diaper. In these embodiments, the mechanical fastener is useful for affixing the absorbent pad to the top sheet of the adult incontinence article, and adequate shear is useful for holding the pad in place while the peel strength of the mechanical fastener should be low enough so that the pad may be easily removed by the user or the caregiver. In other embodiments, absorbent pads including the mechanical fastener according to the present disclosure may be attached directly to a user's undergarments for the purpose of urine absorption. It has been conventionally thought that increasing hook density and increasing hook height may be useful for increasing engagement with a loop material (see, e.g., Int. Pat. Appl. Pub. No. WO 2006/101844).

Figure 3 illustrates atypical absorbent article (100) comprising mechanical fastener patches (140) with mechanical fasteners (48) according to the present application. These were obtained by the method of manufacturing mechanical fasteners (48) according to the present application. The absorbent article (100) has a liquid impermeable backsheet (110), a liquid permeable topsheet (120) and an absorbent core (not shown here). The absorbent article (100) furthermore has a front waist region (115) and a rear waist region (125).

Fastening tabs (130) are arranged at the lateral edges of the rear waist region (125) on each side comprising mechanical fastening patches (140) having mechanical fasteners (48). A dedicated landing zone can be arranged on the outer surface of the front waist region (115); alternatively the entire backsheet (110) can comprise a nonwoven material. The surface of the front waste region (115), for example a loop material, knitted material or a non-woven material, can be engaged by the mechanical fasteners (48) of the mechanical fastening patches (140) which are arranged at the fastening tabs (130) for fastening and closing the absorbent article, respectively.

The present disclosure will now be illustrated further by way of examples and illustrative embodiments. However, there is no intention to limit the disclosure to the embodiments illustrated in the examples and the list of embodiments.

### Examples

An ethylene-propylene copolymer resin was extruded through a cast film die onto a rotating mold. The mold had a staggered array of cavities, approximately 0.64 mm apart in cross direction with a density of approximately 273 cavities per square centimeter. Each of the cavities had a cross section in form of a regular hexagon that tapered from the outside surface of the roll towards the end of the cavity. The hexagonal cross section at the outside surface of the roll had a width of approximately 0.5 mm. Width dimension at the distal end of the cavity was approximately 0.2 mm. The cavity had a depth of approx. 0.23 mm. The equipment used differed from that illustrated in Figure 4 in that the resin was pressed into the holes by a second roller along the surface of the mold roller adjacent where the resin was extruded onto the mold. The solidified resin was stripped off from the mold as a web having an array of upstanding stems approximately 0.23 mm long. The obtained web had precursors according the cavity dimensions with a width at the distal end of approximately 0.2 mm and at the proximal end of approximately 0.5 mm. The backing thickness of the precursor web was approximately 0.03 mm. The basis weight of the web was approximately 60 g/m².

Example 1: To form mechanical fasteners the precursor web was processed by a method as described in U.S. 5,868,987 to form non-circular caps. The resulting fasteners had a height of about 0.21 mm. The oblong cap had an extension in cross direction of approximately 0.38 mm and in machine direction of 0.30 mm.

Example 2: To form mechanical fasteners the precursor web was processed by a similar method as described in Example 1, but with a flat capping roller. The resulting fasteners had a height of approximately 0.2 mm. The capping resulted in a circular cap that had a diameter of approximately 0.33 mm.

Peel and shear properties of these fasteners were evaluated using a nonwoven backsheet. The backsheet has been removed from an adult diaper; Abri Form L2 Super produced by Abena A/S Aabenraa, Denmark.

For peel evaluation, a patch of the mechanical fastener (25 mm in the machine direction (MD) and 13 mm in the cross-direction (CD)) was attached to one end of a tape carrier. The remaining open adhesive was covered by a thin nonwoven. From the nonwoven material a patch of 50 mm in the MD and 60 mm in the CD was cut. The mechanical fastener specimen was put on the nonwoven so that in the peel test both materials were evaluated in CD orientation. For engagement of the fasteners the hook specimen was pressed onto the loop materials using one cycle (one cycle = one forward and one backward pass) with a 2000-gram roller moving at 300 mm per minute. After clamping the nonwoven loop in the lower jaw and the hook specimen in the upper jaw T-peel is tested at a separation speed of 300 mm per minute.

For the example 1 and 2 maximum peel strength were 1.4 N and 1.3 N.

For shear evaluation, a patch of the mechanical fastener (25 mm in the machine direction (MD) and 13 mm in the cross-direction (CD)) was attached to one end of a tape carrier. The remaining open adhesive was covered by a thin nonwoven. The nonwoven material (50 mm in the MD and 60 mm in the CD) was attached with double sided tape to a metal plate. The mechanical fastener specimen was put on the nonwoven so that in the shear test both materials were evaluated in CD orientation. The mechanical fastener specimen was pressed onto the nonwoven using one cycle (one cycle = one forward and one backward pass) with a 2000-gram roller moving at 300 mm per minute. After clamping the metal plate with the nonwoven loop in the lower jaw and the hook specimen in the upper jaw shear is tested at a separation speed of 300 mm per minute.

In examples 1 and 2 maximum peels were 6 N and 11 N.

These examples show that precursors with their specific broad but flat geometry can still be removed from molds even with thin backings. Hooks prepared from the precursors according to the present description have similar peel and shear force behavior than commercial hooks despite their broader and flatter geometries.

## Claims

1. A method of making a web (40) comprising:
- providing a web (10) of precursors (18) for mechanical fasteners (48), the web (10) comprising at least one backing layer (30) and a plurality of upstanding precursors (18) for mechanical fasteners arranged on the backing layer (30) and integral with the backing layer (30), wherein each precursor (18) of the plurality of precursors has a distal end (12) and a proximal end (16) by which the precursor (18) is connected to the backing layer (30), and wherein the base width (22) at the proximal end (16) of the precursor is greater than the height (24) of the precursor (18), and wherein the precursors (18) taper towards their distal ends (12), and wherein the backing layer (30) has a thickness (32) of from about 10 µm to about 75 µm,
- creating a web (40) of mechanical fasteners by subjecting the precursors (18) to shaping to form the fasteners (48), the web (40) comprising at least one backing layer (30) and a plurality of upstanding mechanical fasteners (48) arranged on the backing layer (30) and integral with the backing layer (30), wherein each of the mechanical fasteners (48) of that plurality has (i) head portion (42) comprising the distal end (44) of the fastener (48) and (ii) a stem portion linking the head portion (42) of the fastener (48) with the backing layer (30) and comprising the proximal end (50) by which the fastener (48) is linked with the backing layer (30), wherein the stem portion is tapered and the width (52) of the head portion (42) is greater than the width (58) of the stem immediately underneath the head portion (42) such that the head portion (42) forms an overhang and wherein the width (52) of the head portion (42) is equal or less than the base width (54) of the fastener (48) at the proximal end (50) and wherein the base width (54) is greater than their height (26) of the fastener (48), and wherein the thickness (32) of the backing layer (30) is from about 10µm up to about 75 µm,
wherein head portions of the upstanding precursors (18) of the web of precursors (18) are deformed using heat and/or pressure,
wherein deforming comprises contacting distal tips of the upstanding precursors (18) of the web of precursors (18) with a heated surface and/or contacting the distal tips of the upstanding precursors (18) with a surface that is not heated,
wherein if the surface is not heated, the defomation is carried out with pressure and without heating,
wherein the head portion forms an overhang on each side of the stem.

2. Method according to claim 1, wherein the backing layer (30) and the precursors (18) comprise the same thermoplastic material.

3. Method according to any one of the preceding claims, wherein the precursors (18) have a height (24) of from about 100 and up to about 1000 µm.

4. Method according to any one of the preceding claims, wherein the precursors have a width (22) from about 105 µm and up to about 1010 µm.

5. Method according to any one of the preceding claims, wherein the precursors (18) have a ratio of base width (22) to height (24) of from about 1.1:1.0 to 10:1.

6. Method according to claim 1, wherein the fasteners (48) have a height (26) of from about 90 up to about 900 µm.

7. Method according to claim 1, wherein the fasteners (48) have a base width (54) from about 105 µm to about 1010µm.

8. Method according to claim 1, wherein the fasteners (48) have a ratio of width (52) of head portion to base width (54) is from about 1.0:1.1 to about 1 to 5.

9. Method according to claim 1, wherein the fasteners (48) have a ratio of base width (54) to height (26) of from about 1.2:1.0 to about 10.5:1.0.

## Patentansprüche

1. Verfahren zum Herstellen einer Bahn (40), umfassend:
- Bereitstellen einer Bahn (10) von Vorläufern (18) für mechanische Befestigungselemente (48), die Bahn (10) umfassend mindestens eine Trägerschicht (30) und eine Vielzahl von aufrechten Vorläufern (18) für mechanische Befestigungselemente, die an der Trägerschicht (30) angeordnet und mit der Trägerschicht (30) einstückig verbunden sind, wobei jeder Vorläufer (18) der Vielzahl von Vorläufern ein distales Ende (12) und ein proximales Ende (16) aufweist, durch welches der Vorläufer (18) mit der Trägerschicht (30) verbunden ist, und wobei die Basisbreite (22) an dem proximalen Ende (16) des Vorläufers größer als die Höhe (24) des Vorläufers (18) ist, und wobei die Vorläufer (18) sich zu ihren distalen Enden (12) hin verjüngen, und wobei die Trägerschicht (30) eine Dicke (32) von etwa 10 µm bis etwa 75 µm aufweist,
- Erzeugen einer Bahn (40) von mechanischen Befestigungselementen, indem die Vorläufer (18) einer Formung unterzogen werden, um die Befestigungselemente (48) auszubilden, die Bahn (40) umfassend mindestens eine Trägerschicht (30) und eine Vielzahl von aufrechten mechanischen Befestigungselementen (48), die an der Trägerschicht (30) angeordnet und mit der Trägerschicht (30) einstückig verbunden sind, wobei jedes der mechanischen Befestigungselemente (48) dieser Vielzahl (i) einen Kopfabschnitt (42), umfassend das distale Ende (44) des Befestigungselements (48), und (ii) einen Schaftabschnitt aufweist, der den Kopfabschnitt (42) des Befestigungselements (48) mit der Trägerschicht (30) verbindet und das proximale Ende (50) umfasst, durch das das Befestigungselement (48) mit der Trägerschicht (30) verbunden ist, wobei der Schaftabschnitt verjüngt ist und die Breite (52) des Kopfabschnitts (42) größer als die Breite (58) des Schafts unmittelbar unter dem Kopfabschnitt (42) ist, sodass der Kopfabschnitt (42) einen Überhang ausbildet, und wobei die Breite (52) des Kopfabschnitts (42) gleich oder kleiner als die Basisbreite (54) des Befestigungselements (48) an dem proximalen Ende (50) ist und wobei die Basisbreite (54) größer als ihre Höhe (26) des Befestigungselements (48) ist, und wobei die Dicke (32) der Trägerschicht (30) von etwa 10 µm bis etwa 75 µm beträgt,
wobei Kopfabschnitte der aufrechten Vorläufer (18) der Bahn von Vorläufern (18) unter Verwendung von Wärme und/oder Druck verformt werden,
wobei das Verformen das Kontaktieren von distalen Spitzen der aufrechten Vorläufer (18) der Bahn von Vorläufern (18) mit einer erwärmten Oberfläche und/oder das Kontaktieren der distalen Spitzen der aufrechten Vorläufer (18) mit einer Oberfläche, die nicht erwärmt wird, umfasst,
wobei, falls die Oberfläche nicht erwärmt wird, die Verformung mit Druck und ohne Erwärmen durchgeführt wird,
wobei der Kopfabschnitt auf jeder Seite des Schafts einen Überhang ausbildet.

2. Verfahren nach Anspruch 1, wobei die Trägerschicht (30) und die Vorläufer (18) dasselbe thermoplastische Material umfassen.

3. Verfahren nach einem der vorstehenden Ansprüche, wobei die Vorläufer (18) eine Höhe (24) von etwa 100 und bis zu etwa 1000 µm aufweisen.

4. Verfahren nach einem der vorstehenden Ansprüche, wobei die Vorläufer eine Breite (22) von etwa 105 µm und bis zu etwa 1010 µm aufweisen.

5. Verfahren nach einem der vorstehenden Ansprüche, wobei die Vorläufer (18) ein Verhältnis von der Basisbreite (22) zu der Höhe (24) von etwa 1,1 : 1,0 bis 10 : 1 aufweisen.

6. Verfahren nach Anspruch 1, wobei die Befestigungselemente (48) eine Höhe (26) von etwa 90 bis etwa 900 µm aufweisen.

7. Verfahren nach Anspruch 1, wobei die Befestigungselemente (48) eine Basisbreite (54) von etwa 105 µm bis etwa 1010 µm aufweisen.

8. Verfahren nach Anspruch 1, wobei die Befestigungselemente (48) ein Verhältnis von der Breite (52) des Kopfabschnitts zu der Basisbreite (54) von etwa 1,0 : 1,1 bis etwa 1 zu 5 aufweisen.

9. Verfahren nach Anspruch 1, wobei die Befestigungselemente (48) ein Verhältnis von der Basisbreite (54) zu der Höhe (26) von etwa 1,2 : 1,0 bis etwa 10,5 : 1,0 aufweisen.

## Revendications

1. Procédé de fabrication d'une bande (40), comprenant :
- la fourniture d'une bande (10) de précurseurs (18) pour des moyens de fixation mécaniques (48), la bande (10) comprenant au moins une couche de renfort et de support (30) et une pluralité de précurseurs dressés à la verticale (18) pour les moyens de fixation mécaniques qui sont agencés sur la couche de renfort et de support (30) et qui sont d'un seul tenant avec la couche de renfort et de support (30), dans lequel chaque précurseur (18) de la pluralité de précurseurs comporte une extrémité distale (12) et une extrémité proximale (16) au moyen de laquelle le précurseur (18) est connecté à la couche de renfort et de support (30), et dans lequel la largeur de base (22) au niveau de l'extrémité proximale (16) du précurseur est plus grande que la hauteur (24) du précurseur (18), et dans lequel les précurseurs (18) sont profilés selon une forme conique en direction de leurs extrémités distales (12), et dans lequel la couche de renfort et de support (30) présente une épaisseur (32) comprise entre environ 10 µm et environ 75 µm ;
- la création d'une bande (40) de moyens de fixation mécaniques en soumettant les précurseurs (18) à une mise en forme de manière à former les moyens de fixation mécaniques (48), la bande (40) comprenant au moins une couche de renfort et de support (30) et une pluralité de moyens de fixation mécaniques dressés à la verticale (48) qui sont agencés sur la couche de renfort et de support (30) et qui sont d'un seul tenant avec la couche de renfort et de support (30), dans lequel chacun des moyens de fixation mécaniques (48) de ladite pluralité comporte (i) une partie de tête (42) qui comprend l'extrémité distale (44) du moyen de fixation mécanique (48) et (ii) une partie de tige qui lie la partie de tête (42) du moyen de fixation mécanique (48) avec la couche de renfort et de support (30) et qui comprend l'extrémité proximale (50) au moyen de laquelle le moyen de fixation mécanique (48) est lié avec la couche de renfort et de support (30), dans lequel la partie de tige est profilée selon une forme conique et la largeur (52) de la partie de tête (42) est plus grande que la largeur (58) de la tige immédiatement au-dessous de la partie de tête (42) de telle sorte que la partie de tête (42) forme une partie en saillie et dans lequel la largeur (52) de la partie de tête (42) est égale ou inférieure à la largeur de base (54) du moyen de fixation mécanique (48) au niveau de l'extrémité proximale (50) et dans lequel la largeur de base (54) est plus grande que la hauteur (26) du moyen de fixation mécanique (48) et dans lequel l'épaisseur (32) de la couche de renfort et de support (30) est comprise entre environ 10 µm et environ 75 µm ;
dans lequel les parties de tête des précurseurs dressés à la verticale (18) de la bande de précurseurs (18) sont déformées en utilisant de la chaleur et/ou une pression ;
dans lequel la déformation comprend la mise en contact de pointes distales des précurseurs dressés à la verticale (18) de la bande de précurseurs (18) avec une surface chauffée et/ou la mise en contact des pointes distales des précurseurs dressés à la verticale (18) avec une surface qui n'est pas chauffée ;
dans lequel, si la surface n'est pas chauffée, la déformation est mise en œuvre au moyen d'une pression et sans chauffage ; et
dans lequel la partie de tête forme une partie en saillie sur chaque côté de la tige.

2. Procédé selon la revendication 1, dans lequel la couche de renfort et de support (30) et les précurseurs (18) sont constitués à partir du même matériau thermoplastique.

3. Procédé selon l'une quelconque des revendications précédentes, dans lequel les précurseurs (18) présentent une hauteur (24) comprise entre environ 100 µm et environ 1 000 µm.

4. Procédé selon l'une quelconque des revendications précédentes, dans lequel les précurseurs présentent une largeur (22) comprise entre environ 105 µm et environ 1 010 µm.

5. Procédé selon l'une quelconque des revendications précédentes, dans lequel les précurseurs (18) présentent un rapport largeur de base (22) sur hauteur (24) compris entre environ 1,1:1,0 et 10:1.

6. Procédé selon la revendication 1, dans lequel les moyens de fixation mécaniques (48) présentent une hauteur (26) comprise entre environ 90 µm et environ 900 µm.

7. Procédé selon la revendication 1, dans lequel les moyens de fixation mécaniques (48) présentent une largeur de base (54) comprise entre environ 105 µm et environ 1 010 µm.

8. Procédé selon la revendication 1, dans lequel les moyens de fixation mécaniques (48) présentent un rapport largeur (52) de partie de tête sur largeur de base (54) compris entre environ 1,0:1,1 et environ 1 à 5.

9. Procédé selon la revendication 1, dans lequel les moyens de fixation mécaniques (48) présentent un rapport largeur de base (54) sur hauteur (26) compris entre environ 1,2:1,0 et environ 10,5:1,0.
